# EUROPÄISCHE PATENTSCHRIFT

(11) **EP 2 355 749 B1**
(45) Veröffentlichungstag und Bekanntmachung des Hinweises auf die Patenterteilung: **17.02.2016**
(21) Anmeldenummer: 09783111.9
(22) Anmeldetag: 17.09.2009
(51) Int. Cl.: A61F 2/34, A61F 2/00, A61F 2/30

(54) **HÜFTGELENKPFANNE MIT NICHTMETALLISCHER ABDECKUNG AM UNTEREN RAND DER AUSSENSCHALE**
HIP JOINT SOCKET WITH NON-METALLIC COVER AT THE LOWER EDGE OF THE OUTER SHELL
CAVITÉ COTYLOÏDE AVEC RECOUVREMENT NON MÉTALLIQUE SUR LE BORD INTÉRIEUR DE LA COQUE EXTÉRIEURE

(30) Priorität: 20.11.2008 DE 102008058153
(43) Veröffentlichungstag der Anmeldung: 17.08.2011
(73) Patentinhaber: Aesculap AG, 78532 Tuttlingen (DE)
(72) Erfinder: HOLTMANN, Miriam, 78609 Tuningen (DE); LOHRMANN, Elke, 50739 Köln (DE)
(74) Vertreter: Hoeger, Stellrecht & Partner Patentanwälte mbB
(86) Internationale Anmeldenummer: PCT/EP2009/062044
(87) Internationale Veröffentlichungsnummer: WO 2010/057699

(56) Entgegenhaltungen:
- EP-A- 0 313 762
- FR-A- 2 635 968
- US-A- 5 263 988
- US-A1- 2005 267 585

## Beschreibung

Die Erfindung betrifft eine Hüftgelenkpfanne zur Implantation in den Beckenknochen mit einer Außenschale aus Metall und mit einem in dieser angeordneten Einsatz zur Lagerung einer Gelenkkugel eines Femurimplantates.

Bei einer hüftendprothetischen Versorgung, bei der sowohl eine Hüftgelenkpfanne mit Einsatz als auch ein Femurimplantat mit Schaft und Gelenkkopf implantiert werden, wird der Gelenkkopf bei der Reponierung in sehr kräftiger Anlage am unteren Rand der Außenschale entlang geführt, und dies kann dazu führen, dass sich auf der Gelenkkugel Metall ablagert, das die Gelenkkugel vom Rand der metallischen Außenschale beim Gleiten über diesen Rand abträgt. Dies kann besonders dann auftreten, wenn die Gelenkkugel aus einem sehr harten Material besteht, beispielsweise aus Keramik oder aus einer Kobalt-Chrom-Legierung, während die Außenschale in üblicher Weise aus Titan oder einer Titanlegierung besteht. Es treten dann auf den Gelenkköpfen nach dem Reponieren Titanaufträge auf, und diese metallischen Aufträge auf dem Gelenkkopf können zu einem erhöhten Abrieb des Einsatzmaterials führen, mit dem der Gelenkkopf im eingesetzten Zustand zusammenwirkt.

Aus US 2005/0267585 ist eine Hüftgelenkpfanne mit den Merkmalen des Oberbegriffs von Anspruch 1 bekannt.

Es ist Aufgabe der Erfindung, einen solchen metallischen Auftrag auf der Gelenkkugel und damit einen dadurch bedingten erhöhten Abrieb des Einsatzmateriales zu verhindern.

Diese Aufgabe wird bei einer Hüftgelenkpfanne der eingangs beschriebenen Art erfindungsgemäß dadurch gelöst, dass der untere Rand der Außenschale entsprechend Anspruch 1 von einem nichtmetallischen Material abgedeckt ist. Diese Ausgestaltung verhindert einen unmittelbaren Kontakt der Gelenkkugel mit dem metallischen Material der Außenschale beim Reponieren, und damit ist auch sichergestellt, dass kein Material der Außenschale auf der Gelenkkugel abgelagert werden kann.

Es kann vorgesehen werden, dass sich das nichtmetallische Material auch über den unteren Rand des Einsatzes erstreckt, unbedingt notwendig ist dies jedoch nicht.

Das nichtmetallische Material kann den unteren Rand der Außenschale in unterschiedlicher Weise abdecken.

Beispielsweise kann vorgesehen sein, dass das nichtmetallische Material die Form einer Folie hat, insbesondere einer Folie, die vom unteren Rand der Außenschale und gegebenenfalls des Einsatzes abziehbar ist.

Erfindungsgemäss ist vorgesehen, dass das nichtmetallische Material die Form einer Beschichtung des unteren Randes der Außenschale und gegebenenfalls des Einsatzes hat.

Bei wieder einer anderen Ausführungsform hat das nichtmetallische Material die Form einer Auflage auf den unteren Rand der Außenschale und gegebenenfalls auf den unteren Rand des Einsatzes.

Dabei kann die Auflage auf dem unteren Rand der Außenschale in unterschiedliche Weise gehalten werden, beispielsweise durch eine Verklebung oder insbesondere mittels einer Klemmverbindung, so dass gegebenenfalls auch eine Ablösung der Auflage von der Außenschale nach erfolgter Reponierung möglich ist.

Bei einer bevorzugten Ausführungsform weist die Auflage einen Träger aus Metall auf, der an seiner dem unteren Rand der Außenschale abgewandten Ende mit dem nichtmetallischen Material beschichtet ist. Der metallische Träger dient der Verbindung der Außenschale einerseits und des nichtmetallischen Materials andererseits.

Bei einer weiteren bevorzugten Ausführungsform hat die Auflage die Form eines zusammendrückbaren Kissens. Dieses kann aus einheitlichem, elastisch zusammendrückbarem Material bestehen, bei einer abgewandelten Ausführungsform weist das Kissen eine Hülle auf, die mit einem fließfähigen Material gefüllt ist.

Es kann vorgesehen sein, dass das nichtmetallische Material ein vom Körper resorbierbares Material ist, so dass nach der Implantation dieses Material allmählich abgebaut und entfernt wird.

Das nichtmetallische Material kann ein fließfähiges Schmiermittel sein, es ist möglich, als nichtmetallisches Material einen Gewebekleber oder Gelatine einzusetzen.

Besonders vorteilhaft ist die Verwendung eines Kunststoffes als nichtmetallisches Material, der vorzugsweise aus der Gruppe der folgenden Materialien ausgewählt ist: Polyethylen, Polyetheretherketon, Polyoxymethylen, Polyphenylsulfon, thermoplastisches Elastomer, Polyurethan, Polytetrafluorethylen.

Die nachfolgende Beschreibung bevorzugter Ausführungsformen der Erfindung dient im Zusammenhang mit der Zeichnung der näheren Erläuterung. Es zeigen:
- Figur 1:: eine in einen Beckenknochen implantierte Hüftgelenkpfanne und ein mit einer Gelenkkugel versehenes Femurimplantat beim Einschieben der Gelenkkugel in den Einsatz der Hüftgelenkpfanne;
- Figur 2:: eine perspektivische Ansicht einer im Schnitt dargestellten Hüftgelenkpfanne mit einer Abdeckung des unteren Randes der Außenschale in Form einer abziehbaren Folie;
- Figur 3:: eine Ansicht ähnlich Figur 2 mit einer Beschichtung des unteren Randes der Außenschale mittels eines Gewebeklebers;
- Figur 4:: eine Ansicht ähnlich Figur 3 mit einer Beschichtung aus Gelatine;
- Figur 5:: eine Ansicht ähnlich Figur 2 mit einer Abdeckung des unteren Randes der Außenschale in Form eines ringförmigen Kissens;
- Figur 6:: eine Ansicht ähnlich Figur 2 mit einer Abdeckung des unteren Randes der Außenschale in Form eines im Klemmsitz an der Außenschale gehaltenen Kunststoffringes und
- Figur 7:: eine Ansicht ähnlich Figur 6 mit einem Abdeckring für den unteren Rand der Außenschale mit einem metallischen, kunststoffbeschichteten Träger.

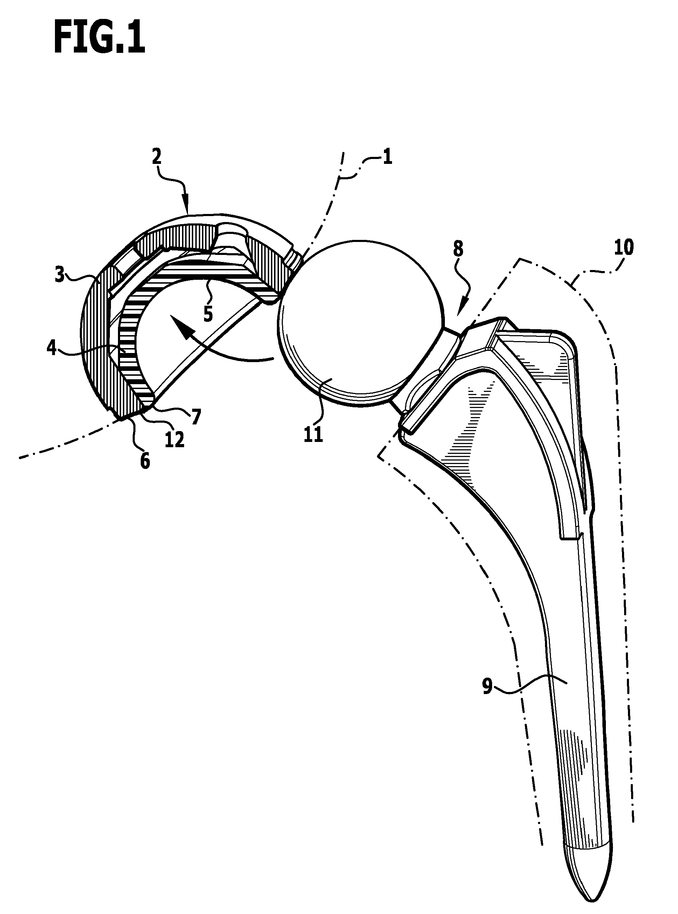

In Figur 1 ist eine in einen Beckenknochen 1 implantierte Hüftgelenkpfanne 2 dargestellt, die eine halbkugelige Außenschale 3 aus Metall aufweist, beispielsweise aus Titan oder einer Titanlegierung, in die ein schalenförmiger Einsatz 4 eingesetzt ist, der ebenfalls im Wesentlichen die Form einer Halbkugel hat und der auf seiner Innenseite eine halbkugelige Lagerfläche 5 ausbildet. An ihrer unteren Kante enden sowohl die Außenschale als auch der Einsatz 4 jeweils in einem unteren Rand 6 beziehungsweise 7, die nebeneinander liegen.

In Figur 1 ist weiterhin ein Femurimplantat 8 dargestellt mit einem Schaft 9, der in einen Femur 10 implantiert ist und der eine außerhalb des Femurs 10 angeordnete Gelenkkugel 11 trägt, deren Außendurchmesser dem Innendurchmesser der Lagerfläche 5 entspricht. Das Femurimplantat 8 besteht üblicherweise aus Metall, beispielsweise aus einer Kobalt-Chrom-Legierung, kann aber auch aus Keramik bestehen.

Nach der Implantation der Hüftgelenkpfanne 2 im Beckenknochen 1 und des Femurimplantates 8 im Femur 10 muss die Gelenkkugel in der Lagerfläche 5 positioniert werden, und dazu ist es notwendig, die Gelenkkugel 11 am unteren Rand 6 der Außenschale 3 und auch am unteren Rand 7 des Einsatzes 4 vorbei zu schieben, wie es aus der Darstellung der Figur 1 ersichtlich ist. Aufgrund der hohen Kräfte des Bandapparates werden dabei die Gelenkkugel 11 und die unteren Ränder 6, 7 der Außenschale 3 beziehungsweise des Einsatzes 4 kräftig gegeneinander gedrückt.

Um dabei zu vermeiden, dass die Gelenkkugel 11 mit dem metallischen Material der Außenschale 3 in Kontakt kommt und von dieser Material mitnimmt, ist der untere Rand 6 der Außenschale 3 und gegebenenfalls auch der untere Rand 7 des Einsatzes 4 von einem nichtmetallischen Material abgedeckt. Dieses verhindert den unmittelbaren Kontakt der Gelenkkugel mit dem metallischen Material der Außenschale 3 und schützt somit sowohl die unteren Ränder der Außenschale 3 und des Einsatzes 4 als auch die Gelenkkugel 11 vor ungewollten Beschädigungen.

Für die Ausgestaltung dieser Abdeckung sind sehr unterschiedliche Möglichkeiten gegeben.

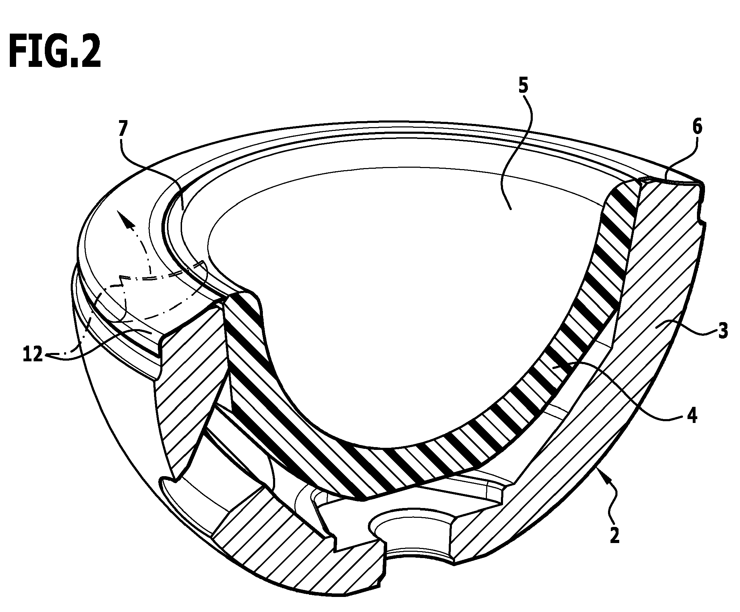

Bei dem Ausführungsbeispiel der Figur 2 wird der untere Rand 6 der Außenschale 3 von einer abziehbaren Folie 12 bedeckt, diese kann mit Hilfe eines Klebstoffes an dem unteren Rand 6 festgelegt sein oder aber auch nur aufgrund von Anziehungskräften zwischen der Folie 12 einerseits und dem unteren Rand 6 andererseits. Nach dem Reponieren der Gelenkkugel 11 in der Lagerfläche 5 kann diese ringförmige Folie vom unteren Rand 6 abgezogen werden.

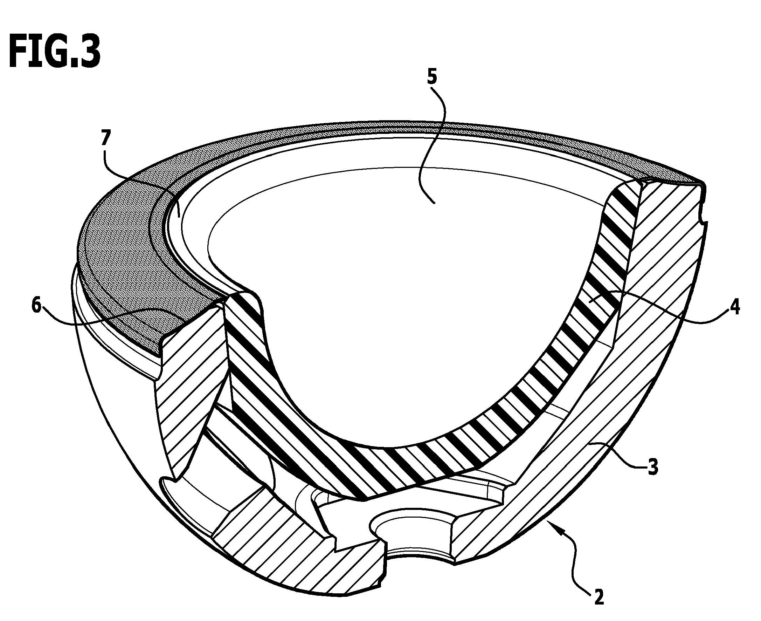

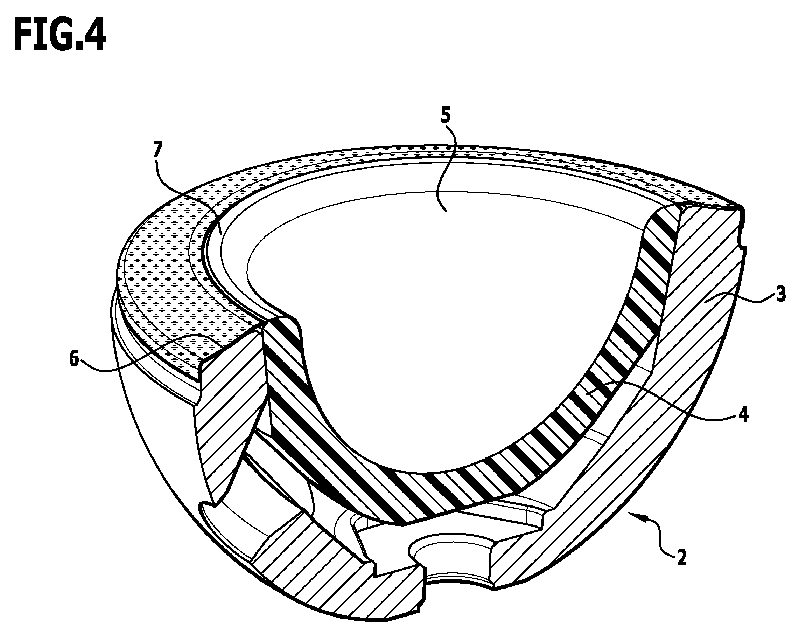

Es ist auch möglich, wie dies in Figur 3 dargestellt ist, den unteren Rand 6 mit einer nichtmetallischen Substanz zu beschichten, beispielsweise mittels eines biokompatiblen Schmiermittels, wie zum Beispiel Silikonöl oder dickflüssige Gelatine, wie dies in Figur 3 dargestellt ist, oder mittels Gelatine, wie dies in Figur 4 dargestellt ist. Vorzugsweise sind diese Materialien vom Körper resorbierbar, so dass sie nach erfolgter Implantation allmählich abgebaut und resorbiert werden.

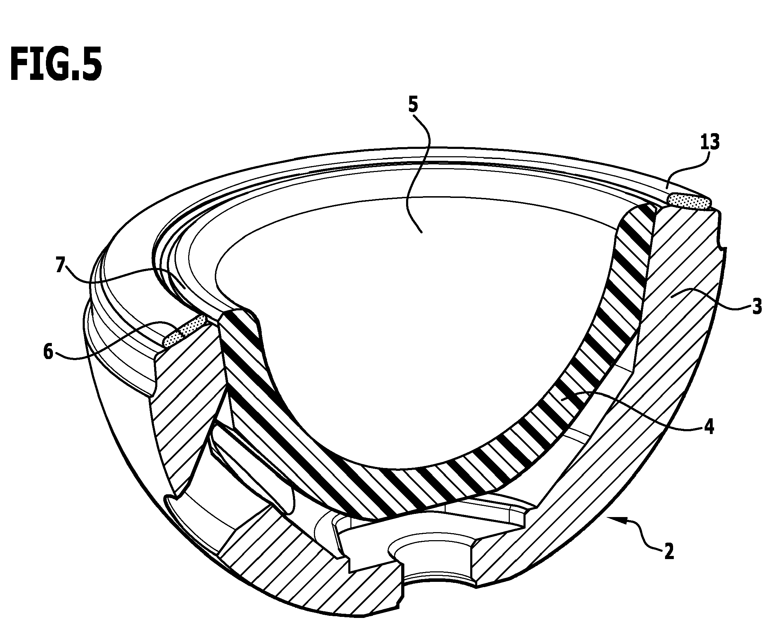

Bei dem Ausführungsbeispiel der Figur 5 ist auf den unteren Rand 6 ein ringförmiges Kissen 13 aufgelegt, dieses kann aus einem komprimierbaren Material bestehen, beispielsweise aus einem Hydrogel, aus Silikon oder aus einem thermoplastischen Elastomer, es ist auch möglich, dass dieses Kissen 13 eine Außenhülle aufweist, die mit einem fließfähigen Material gefüllt ist, beispielsweise einer NaCl-Lösung. Auch dieses Kissen 13 kann ablösbar am unteren Rand 6 gehalten sein.

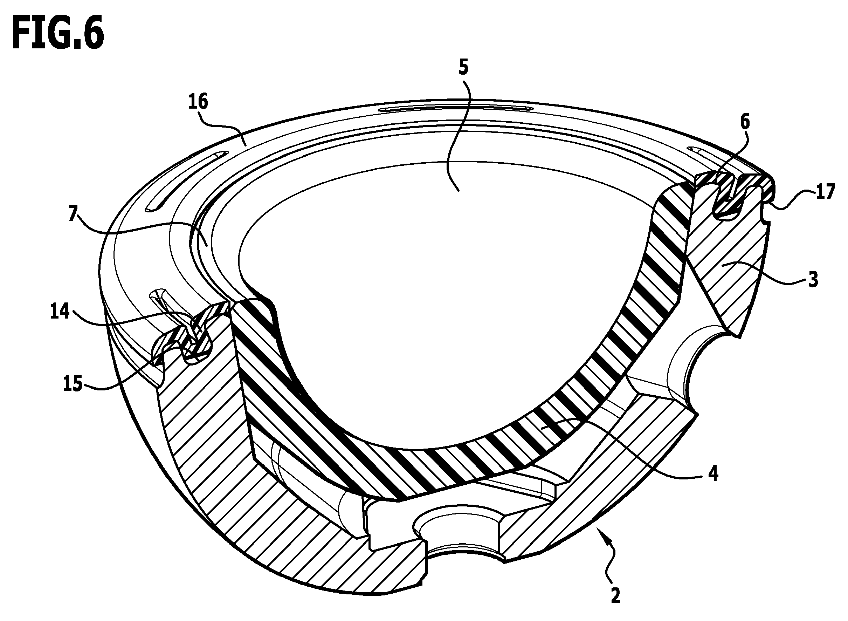

Bei dem Ausführungsbeispiel der Figur 6 ist auf den unteren Rand 6 ein Kunststoffring 16 abdeckend aufgelegt, der mittels mehrer Klemmvorsprünge 14 an dem unteren Rand 6 klemmend festgelegt ist, diese Klemmvorsprünge 14 tauchen in Vertiefungen 15 des unteren Randes 6 ein. Die Klemmwirkung wird außerdem verstärkt durch seitlich umgebogene, den unteren Rand 6 der Außenschale 3 umgreifende Ränder 17 des Kunststoffringes 16.

Auch dieser Kunststoffring 16 kann gegebenenfalls unter Lösung der Klemmverbindung von der Außenschale 3 abgezogen werden, es ist aber auch möglich, dass dieser Kunststoffring 16 nach der Implantation verbleibt, insbesondere kann er aus einem resorbierbaren Kunststoffmaterial bestehen, welches vom Körper allmählich zersetzt und abgebaut wird.

Mögliche Materialien für einen solchen Kunststoffring 16 sind beispielsweise Polyethylen, Polyetheretherketon, Polyoxymethylen, Polyphenylsulfon, thermoplastisches Elastomer, Polyurethan oder Polytetrafluorethylen.

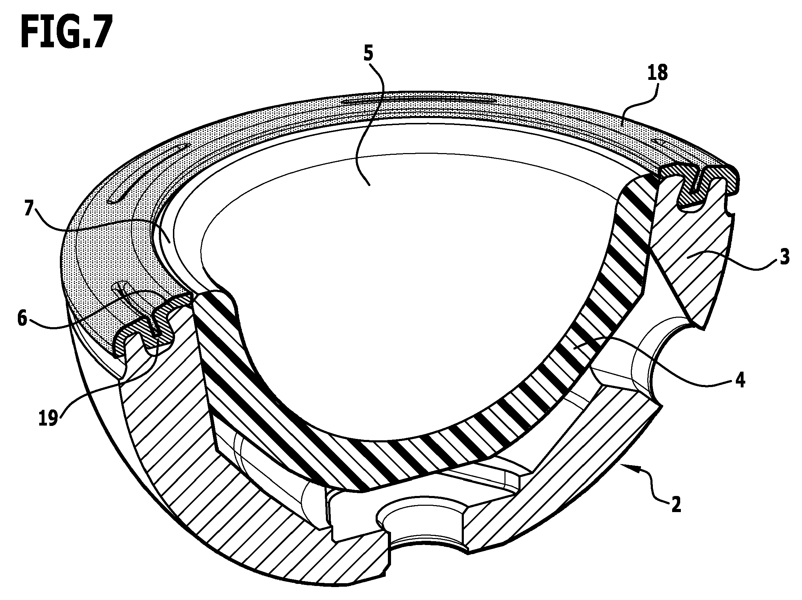

Während bei dem Ausführungsbeispiel der Figur 6 die Abdeckung durch einen Ring erfolgt, der vollständig aus Kunststoff besteht, ist im Ausführungsbeispiel der Figur 7 zur Abdeckung ein ähnlich geformter Abdeckring 18 vorgesehen, der aus einem metallischen Träger 19 besteht und auf seiner von der Außenschale 3 abgewandten Seite mit einem Kunststoffmaterial beschichtet ist. Es kann sich dabei um dieselben Kunststoffmaterialien handeln, die vorstehend aufgeführt sind.

## Patentansprüche

1. Hüftgelenkpfanne (2) zur Implantation in den Beckenknochen (1) mit einer Außenschale (3) aus Metall und mit einem in dieser angeordneten Einsatz (4) zur Lagerung einer Gelenkkugel (11) eines Femurimplantates (8), bei welcher der untere Rand (6) der Außenschale (3) von einem nichtmetallischen Material (12; 13; 16; 18) abgedeckt ist, **dadurch gekennzeichnet, dass** das nichtmetallische Material die Form einer Beschichtung des unteren Randes (6) der Außenschale (3) hat.

2. Hüftgelenkpfanne nach Anspruch 1, **dadurch gekennzeichnet, dass** sich die Beschichtung durch das nichtmetallische Material auch über den unteren Rand (7) des Einsatzes (4) erstreckt.

3. Hüftgelenkpfanne nach einem der voranstehenden Ansprüche, **dadurch gekennzeichnet, dass** das nichtmetallische Material ein vom Körper resorbierbares Material ist.

4. Hüftgelenkpfanne nach einem der voranstehenden Ansprüche, **dadurch gekennzeichnet, dass** das nichtmetallische Material ein fließfähiges Schmiermittel ist.

5. Hüftgelenkpfanne nach einem der Ansprüche 1 bis 3, **dadurch gekennzeichnet, dass** das nichtmetallische Material ein Gewebekleber ist.

6. Hüftgelenkpfanne nach einem der Ansprüche 1 bis 3, **dadurch gekennzeichnet, dass** das nichtmetallische Material Gelatine ist.

7. Hüftgelenkpfanne nach einem der Ansprüche 1 bis 3, **dadurch gekennzeichnet, dass** das nichtmetallische Material ein Kunststoff aus der Gruppe Polyethylen, Polyetheretherketon, Polyoxymethylen, Polyphenylsulfon, thermoplastisches Elastomer, Polyurethan, Polytetrafluorethylen ist.

## Claims

1. Hip joint socket (2) for implantation into the pelvic bone (1), comprising an outer shell (3) consisting of metal and an insert (4) arranged in it for supporting a joint ball (11) of a femur implant (8), the lower edge (6) of the outer shell (3) of said hip joint socket being covered by a non-metallic material (12; 13; 16; 18), **characterized in that** the non-metallic material has the form of a coating of the lower edge (6) of the outer shell (3).

2. Hip joint socket as defined in claim 1, **characterized in that** the coating by the non-metallic material also extends over the lower edge (7) of the insert (4).

3. Hip joint socket according to any one of the preceding claims, **characterized in that** the non-metallic material is a material resorbable by the body.

4. Hip joint socket according to any one of the preceding claims, **characterized in that** the non-metallic material is a flowable lubricating material.

5. Hip joint socket as defined in claims 1 to 3, **characterized in that** the non-metallic material is a tissue adhesive.

6. Hip joint socket as defined in claims 1 to 3, **characterized in that** the non-metallic material is gelatin.

7. Hip joint socket as defined in claims 1 to 3, **characterized in that** the non-metallic material is a plastic material from the group consisting of polyethylene, polyether ether ketone, polyoxymethylene, polyphenylsulfone, thermoplastic elastomers, polyurethane, polytetrafluoroethylene.

## Revendications

1. Cotyle de la hanche, destinée à l'implantation dans l'os du bassin (1), et comprenant une coque extérieure (3) en métal dans laquelle est agencé un insert (4) destiné à assurer le montage d'une rotule d'articulation (11) d'un implant fémoral (8), le bord inférieur (6) de la coque extérieure (3) étant recouvert d'un matériau (12; 13; 16; 18) non métallique, **caractérisée en ce que** le matériau non métallique se présente sous la forme d'un revêtement du bord inférieur (6) de la coque extérieure (3).

2. Cotyle de la hanche selon la revendication 1, **caractérisée en ce que** le revêtement par le matériau non métallique s'étend également par-dessus le bord inférieur (7) de l'insert (4).

3. Cotyle de la hanche selon l'une des revendications précédentes, **caractérisée en ce que** le matériau non métallique est un matériau résorbable par le corps.

4. Cotyle de la hanche selon l'une des revendications précédentes, **caractérisée en ce que** le matériau non métallique est un agent lubrifiant susceptible de s'écouler.

5. Cotyle de la hanche selon l'une des revendications 1 à 3, **caractérisée en ce que** le matériau non métallique est une colle tissulaire.

6. Cotyle de la hanche selon l'une des revendications 1 à 3, **caractérisée en ce que** le matériau non métallique est de la gélatine.

7. Cotyle de la hanche selon l'une des revendications 1 à 3, **caractérisée en ce que** le matériau non métallique est une matière plastique du groupe polyéthylène, polyétheréthercétone, polyoxyméthylène, polyphénylsulfone, élastomère thermoplastique, polyuréthanne, polytétrafluoroéthylène.
